# EUROPEAN PATENT SPECIFICATION

(11) **EP 2 731 671 B1**
(45) Date of publication and mention of the grant of the patent: **03.04.2019**
(21) Application number: 12821988.8
(22) Date of filing: 11.07.2012
(51) Int. Cl.: A61N 1/36, A61N 1/362, A61B 18/14, A61N 1/05

(54) **CATHETER SYSTEM FOR ACUTE NEUROMODULATION**
KATHETERSYSTEM FÜR AKUTE NEUROMODULATION
SYSTÈME DE CATHÉTER POUR NEUROMODULATION AIGUË

(30) Priority: 11.07.2011 US 201161506164 P; 25.10.2011 US 201161551418 P; 09.01.2012 US 201261584812 P; 21.02.2012 US 201261601501 P; 20.03.2012 US 201261613433 P; 29.04.2012 US 201261639982 P
(43) Date of publication of application: 21.05.2014
(73) Proprietor: INTERVENTIONAL AUTONOMICS CORPORATION, Durham, NC 27703 (US)
(72) Inventor: RANSBURY, Terrance, Chapel Hill, NC 27517 (US); SANDERS, William, E., Chapel Hill, NC 27517 (US); STACK, Richard, S., Chapel Hill, NC 17517 (US); MASSON, Stephen, C., Chapel Hill, NC 27517 (US)
(74) Representative: Millburn, Julie Elizabeth
(86) International application number: PCT/US2012/046332
(87) International publication number: WO 2013/022543

(56) References cited:
- WO-A1-2006/115877
- WO-A1-2007/092330
- WO-A2-2012/149511
- US-A1- 2005 288 730
- US-A1- 2006 235 474
- US-A1- 2007 129 760
- US-A1- 2011 022 127
- US-B1- 6 292 695

## Description

### TECHNICAL FIELD OF THE INVENTION

The present application generally relates to systems and methods for acute neuromodulation using stimulation elements disposed within the vasculature.

### BACKGROUND

Acute heart failure syndromes (AHFS) are serious conditions resulting in millions of hospitalizations each year. AHFS treatments can include pharmacologic inotrope administration - however side effects of such treatments, including arrhythmias and increased myocardial oxygen demand, can contribute to patient mortality. Additional treatments include administration of diuretics to treat pulmonary edema resulting from AHFS.

The autonomic nervous system includes the parasympathetic nervous system and the sympathetic nervous system. The parasympathetic and sympathetic nervous system have somewhat opposing effects on the cardiovascular system. One function of the parasympathetic nervous system is to slow the heart through action of the vagus nerve. On the other hand, the sympathetic nervous system is associated with increasing the heart rate and increasing the contractility of the heart. The disclosed system and method may be used to augment balance between the sympathetic and parasympathetic systems in AHFS patents so as to lower heart rate, elevate heart rate and/or increase heart contractility.

US 2005/288730 A1 discloses apparatus for renal neuromodulation comprising: a pulsed electric field generator; and a device configured for percutaneous placement proximate a renal vasculature in a patient, wherein the device comprises an electrode electrically coupled to the pulsed electric field generator for delivering a pulsed electric field to renal nerves while the device is located proximate the renal vasculature.

US 2006/0235474 A1 discloses apparatus for performing renal neuromodulation of a patient comprising: a pulsed electric field generator; a first element configured for placement within a first vessel of the patient, the first element comprising a first electrode electrically coupled to the pulsed electric field generator; and a second element configured for placement within a second vessel of the patient that is different than the first vessel, the second element comprising a second electrode electrically coupled to the pulsed electric field generator, wherein the apparatus is configured for delivery of a pulsed electric field between the first electrode and the second electrode to modulate a neural fiber that contributes to renal function while the first device is located within the first vessel and the second device is located within the second vessel.

The invention is defined in claim 1. Further aspects and preferred embodiments are defined in the dependent claims. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention.

### BRIEF DESCRIPTION OF THE DRAWINGS

Figs. 1-4 are a sequence of drawings illustrating deployment of a first embodiment of a catheter system, in which:
   Fig. 1 shows the system in a blood vessel prior to expansion of the anchoring element;
   Fig. 2 is similar to Fig. 1 but shows the anchoring element expanded;
   Fig. 3 illustrates the system following removal of the guide wire and dilator, and
   Fig. 4 is similar to Fig. 3 but shows a Swan-Ganz catheter extending through the lumen of the catheter.
Fig. 5A is similar to Figs. 4 but shows a second neuromodulation device extending through the lumen of the catheter.
Fig. 5B schematically illustrates positioning of the neuromodulation device of Fig. 5A within the vasculature;
Figs. 6 and 7 are similar to Figs. 1 and 4 but show a second alternative configuration for expanding the anchoring element.

### DETAILED DESCRIPTION

The present application discloses a catheter system for neuromodulation. One application of the system is for acute use in treating AHFS through parasympathetic and/or sympathetic neuromodulation. However it should be understood that the system may alternatively be used to treat other conditions, or to maintain autonomic balance at times where the patient's own nervous system could benefit from assistance in maintaining autonomic balance. One example of this latter application is to use the system to maintain autonomic balance while the patient is intubated, is in a coma, or is otherwise experiencing autonomic dysfunction. Other conditions that could be treated with acute neuromodulation include, but are not limited to, acute myocardial infarction, pulmonary embolism, hemorrhage, systemic inflammatory response syndrome (SIRS), sepsis, and post-surgery autonomic dysfunction.

A neuromodulation system for treating AHFS provides therapeutic elements for modulation of parasympathetic and/or sympathetic fibers. In some embodiment, only parasympathetic fibers are stimulated, while in other embodiments parasympathetic and sympathetic fibers are stimulated at the same time and/or at different times to improve autonomic balance in the heart. In preferred embodiments, the therapeutic elements are positioned on one or more catheters positioned in the vasculature of the patient and are energized to modulate nerve fibers positioned outside the vascular walls. Modulation may be carried out to activate and/or inhibit or block activation of target nerve fibers. In the disclosed system, the therapeutic elements are described as electrodes, although it is contemplated that other forms of therapeutic elements (including, but not limited to, ultrasound, thermal, or optical elements) may instead be used.

The parasympathetic and sympathetic fibers may be modulated from the same therapeutic element or element array, or from different elements or element arrays. Elements used to modulate sympathetic fibers may be positioned in the same blood vessels as those used for the parasympathetic fibers, or they may be in different blood vessels. The blood vessel and the target position of the therapeutic elements within a chosen vessel is selected based on the vessel's anatomic location relative to the target fiber so as to position the therapeutic element in close proximity to the target fiber while minimize collateral effects. For example, in the canine model, right sympathetic fibers modulating left ventricular contractility converge at the common pulmonary artery and course in the pulmonary artery nerves. Left sympathetic fibers modulating ventricular contractility are found near the common pulmonary artery, pulmonary artery nerves, and ventral lateral cardiac nerve. In contrast, sympathetic fibers controlling chronotropic and dromotropic functions are found between the superior vena cava (SVC) and aorta, between the common pulmonary artery and the proximal right pulmonary artery, between the left superior pulmonary vein and the right pulmonary artery, and elsewhere. J.L. Ardell et al, Differential sympathetic regulation of automatic, conductile, and contractile tissue in dog heart. The anatomy thus allows a therapeutic element to be positioned to selectively stimulate sympathetic fibers controlling ventricular inotropy to increase contractility, while avoiding chronotropic/dromotropic effects so as not to trigger tachycardia.

In human use, modulation of sympathetic fibers may be achieved using a therapeutic element positioned within the pulmonary artery so as to stimulate sympathetic fibers to increase inotropy. Moreover, therapeutic elements could additionally or alternatively be employed to stimulate parasympathetic fibers that lower heart rate. Such fibers may also be activated using intravascular electrodes located in the pulmonary arteries, although in other embodiments vagal or other parasympathetic fibers are modulated using a therapeutic element in the superior vena cava or the internal jugular vein, preferably on the right side.

In some embodiments, combined or alternating modulation of the parasympathetic and sympathetic fibers may be employed to optimize the opposing effects of parasympathetic and sympathetic modulation on heart rate - such that modulation optimizes the ability of the sympathetic system to drive the heart rate and the parasympathetic system to "apply the brakes" to slow the heart when necessary. Sensed or derived hemodynamic parameters may be used by the system to select and implement stimulation parameters, algorithms and/or to identify the therapeutic element(s) to be activated at a given time. Suitable sensed or derived hemodynamic parameters include pulmonary capillary wedge pressure (PCWP), cardiac index, derivations of vascular resistance, heart rate, and blood pressure (arterial). Other parameters may include central venous pressure, CO/Cl, and cardiac filling pressures.

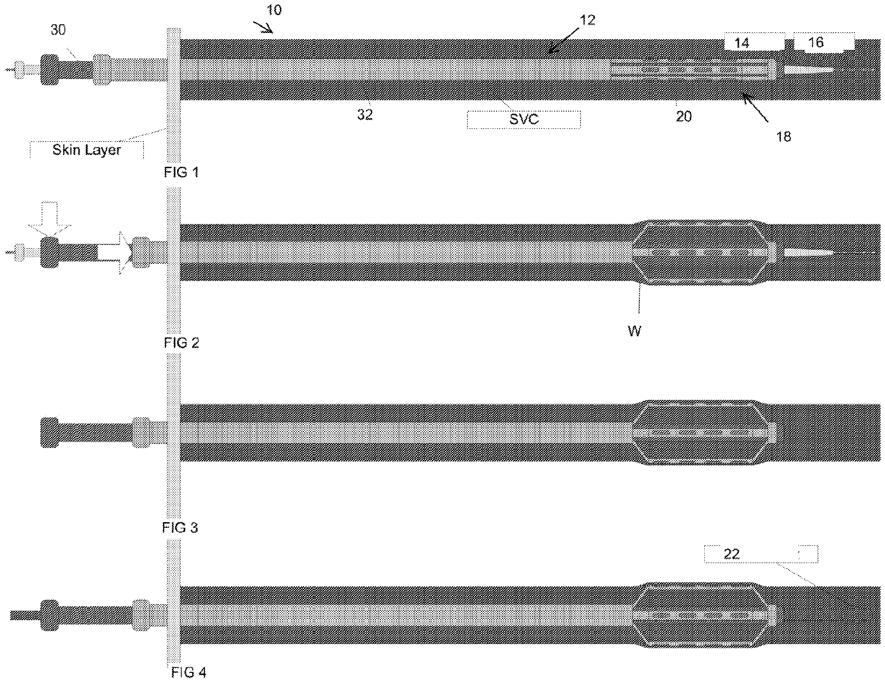

Figs. 1 - 4 illustrate a first embodiment of a catheter system 10, which includes a treatment catheter 12, a dilator 14, and a guide wire 16. The treatment catheter 12 includes a tubular inner sheath 30 and a tubular outer sheath 32, which are connected at their distal end sections.

The distal end section of the outer sheath includes one or more anchoring elements 18 that are expanded or extended into contact with the surrounding vessel wall so as to anchor the catheter in a desired location. The anchoring element(s) may be an expandable basket or stent-like device, or one or more spline elements as illustrated in the drawings. In the illustrated configuration, these elements are outwardly expandable into contact with the vessel wall W when the outer sheath 32 is pushed distally relative to the inner sheath 30 as illustrated in Fig. 2. Since the inner and outer sheaths are connected at their distal end portions, sliding the outer sheath distally relative to the inner sheath causes the anchoring elements to bow outwardly into contact with the vessel wall as shown. Stimulation electrodes 20 are mounted to or formed on the anchoring element(s) 18, or the anchoring element(s) may themselves be configured to function as electrodes. The electrodes are preferably positioned such that expanding the anchoring elements into contact with the vessel wall places the active surfaces of the electrodes into contact with the vessel wall, allowing energy for neuromodulation to conduct from the electrodes through the vessel wall to target nerve fibers adjacent to the vessel (e.g. in the adjacent extravascular space).

The inner sheath 30 includes a lumen, allowing the catheter 12 to function both as a neuromodulation catheter and an introducer for other medical devices useful for the procedure. Examples include catheters for patient monitoring (e.g. Swan-Ganz), additional electrode catheters or leads for a variety of applications such as mapping target stimulation sites, cardiac pacing, or ablation, or catheters/leads carrying neuromodulation electrodes positionable at a second intravascular site to target additional nerve fibers.

In one method of using the first embodiment, a percutaneous Seldinger technique is used to place the guidewire 16 into the venous vasculature, such as via the femoral vein, internal or external jugular vein, or subclavian vein. The dilator 14, which is preferably preloaded into the lumen of the inner sheath 30, is advanced together with the catheter over the wire and directed to the target blood vessel. The user advances the outer sheath 32 relative to the inner sheath 30 (such as by holding the hub of the inner sheath while pushing the hub of the outer sheath distally as shown in Fig. 2) - causing the anchoring elements 18 to expand into contact with the surrounding vessel wall, thus anchoring the catheter at the target site in the vessel and placing the electrodes 20 into contact with the vessel wall. The relative positions of the inner and outer sheath hubs may be locked using a ratchet or locking mechanism (not shown) to maintain the anchoring elements in the expanded position.

The dilator and wire are removed from the catheter lumen either before or after anchoring of the catheter.

In one embodiment, the target vessel is the superior vena cava, and the catheter 12 is anchored such that energizing the electrodes (or a select group of electrodes within the array) will cause a desired effect (e.g. enhance, augment, inhibit or block signaling) on vagus nerve fibers adjacent to the superior vena cava. Once the electrodes are expanded into contact with the vessel wall, mapping procedures may be carried out as known in the art (measuring the effect of stimulus at various electrode locations) to identify the optimal positions of the electrodes or to identify the best combination of electrodes within the array to energize for the desired response.

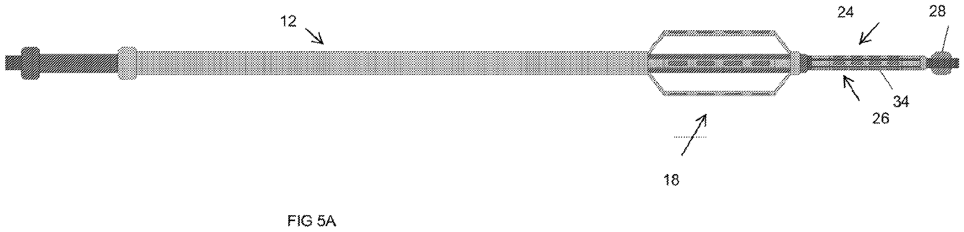

Additional medical devices are advanced through the inner sheath lumen as discussed above, such that their distal portions extend from the distal end of the catheter. Fig. 4 shows use of a Swan-Ganz catheter 22 through the inner sheath 30. Fig. 5A shows that a second electrode lead or catheter 24 can be advanced through the lumen of the inner sheath 30. The second electrode lead or catheter may have one or more expandable anchoring elements 26 as discussed above with respect to the catheter 12 (and as shown in Fig. 5A in the unexpanded position), with electrodes 34 mounted to or formed on the anchoring elements 26 as disclosed. The second electrode lead or catheter 24 may include an inflatable balloon 28 on its distal tip as shown, to facilitate advancement of the second electrode lead/catheter 24 to a target site. It may also include sensing functionality, such as the ability to sense pressures including, but not limited to, PCWP. For example, if the second electrode lead/catheter 24 is to be positioned within the pulmonary artery, inflating the balloon within the right ventricle can help the electrode lead/catheter float with the flowing blood into the pulmonary artery in the manner similar to the way in which a Swan-Ganz catheter is positioned. The balloon 28 may be positioned on the second lead/catheter 34 itself, or on an additional catheter extending through a lumen in the lead/catheter 34.

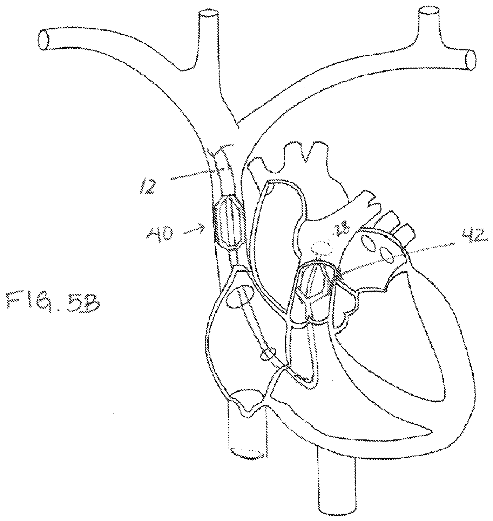

In one exemplary procedure using the Fig. 5A embodiment, the electrodes 20 of the catheter 12 are anchored in the superior vena cava as discussed above for neuromodulating parasympathetic activity of the vagus nerve (to slow the heart, for example), and the electrodes 34 of the second lead/catheter 24 are anchored in the pulmonary artery for directing energy to sympathetic nerves that will enhance heart contractility and/or increase heart rate. Referring to Fig. 5B, in a positioning method according to this embodiment, the catheter is advanced into the superior vena cava and anchoring elements 18 are expanded to position the electrodes 20 against the wall of the SVC, placing the first electrode array 40 in position to stimulate the vagus nerve. Next, the second lead/catheter 24 is further extended from the lumen of the inner sheath 30, and passed or caused to through the right atrium and right ventricle of the heart and into the pulmonary artery using the method described in the prior paragraph or alternative methods. Once in a target position within the pulmonary artery (e.g. pulmonary trunk, or left or right pulmonary artery), the anchoring elements of the second lead/catheter 24 are expanded, positioning the electrodes 34 in apposition with the pulmonary artery wall and thus placing the second electrode array 42 in position to stimulate sympathetic nerves (or, if desired, parasympathetic nerves) in proximity to the pulmonary artery. Pressure may be monitored using pressure transducers on the second lead/catheter, and/or the balloon may be used to monitor pulmonary capillary wedge pressure.

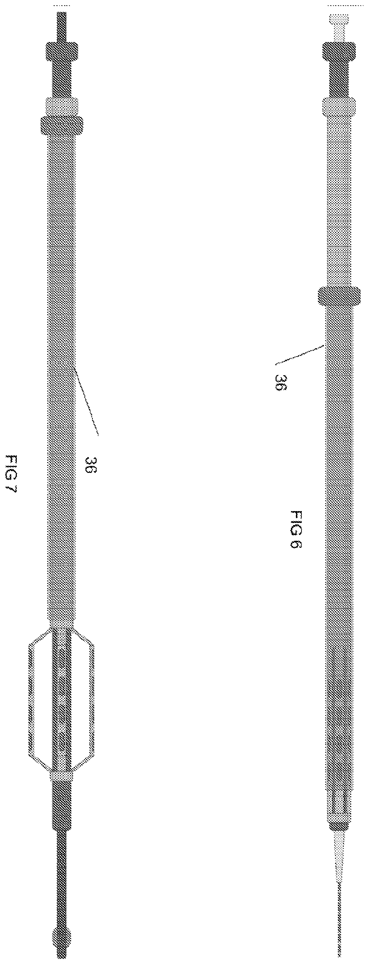

In a slightly modified version of the Fig. 1-4 embodiment, deployment of the anchoring elements 18 is accomplished by pulling the inner sheath 30 proximally relative to the outer sheath 32. Figs. 6-7 show yet another configuration utilizing anchoring elements that are self-expandable upon retraction of an outer sleeve 36 (shown compressing the anchoring elements in Fig. 6 and withdrawn from them in Fig. 7) that maintains the anchoring element(s) in a compressed position until it is retracted. In still other embodiment, pull cables may be tensioned from the proximal end of the catheter to expand the anchoring elements.

The disclosed catheter system may be coupled to external pulse generator used to energize the electrodes using stimulation parameters selected to capture the target nerve fibers and to achieve the desired neuromodulation. Feedback to the pulse generator is provided by one or more diagnostic sensors, including feedback from sensors mounted on or extending through the lumen of the catheter-introducer. The simulation parameters may be determined or adjusted in response to information sensed by the sensors and/or derived from sensor feedback. Suitable sensed or derived hemodynamic parameters include pulmonary capillary wedge pressure (PCWP), cardiac index, derivations of vascular resistance, heart rate, blood pressure (arterial). Other parameters may include central venous pressure, CO/CI, and cardiac filling pressures.

Methods of treating a patient described in this application are defined in the following numbered paragraphs:
1. A method of treating a patient, comprising: (a) stimulating at least one parasympathetic nerve fiber using a first therapeutic element disposed in a superior vena cava of the patient; and (b) stimulating at least one sympathetic nerve fiber using a second therapeutic element disposed in a pulmonary artery of the patient.
2. The method of paragraph 1, further including: introducing a therapy system into the vasculature, the therapy system including the first therapeutic element and the second therapeutic element coupled to the first therapeutic element; advancing the therapy device within the vasculature to position the first therapeutic element within the superior vena cava; and advancing the second therapeutic element relative to the first therapeutic element to position the second therapeutic element within the pulmonary artery.
3. The method of paragraph 1, wherein the first therapeutic element is provided on a first elongate shaft, the second therapeutic element is provided on a second elongate shaft, and wherein advancing the second therapeutic element relative to the first therapeutic element includes sliding a first one of the first and second shafts within a lumen of a second one of the first and second shafts.
4. The method of paragraph 3, wherein advancing the second therapeutic element includes, with the second therapeutic element disposed in the heart, inflating a balloon carried by the second therapeutic element such that the balloon and second therapeutic element are carried by blood flow into the pulmonary artery.
5. The method of paragraph 1, wherein the first therapeutic element comprises electrodes, and wherein step (a) includes energizing the electrodes.
6. The method of paragraph 5, further including anchoring the first therapeutic element within the superior vena cava to bias the electrodes into contact with a wall of the superior vena cava.
7. The method of paragraph 1, wherein the second therapeutic element comprises electrodes, and wherein step (b) includes energizing the electrodes.
8. The method of paragraph 7, further including anchoring the second therapeutic element within the pulmonary artery to bias the electrodes into contact with a wall of the pulmonary artery.
9. The method of paragraph 1, further including sensing at least one parameter within the body using the therapy device.
10. The method of paragraph 9, wherein the sensed parameter comprises pressure.

## Claims

1. A neuromodulation system (10) for treating a patient, comprising:
a first therapy element adapted for positioning within a first blood vessel, the first therapy element carried on a first elongate shaft (12), wherein the first shaft includes:
an outer sheath (32) having a distal portion and an expandable anchoring element (18) on the distal portion, wherein the first therapy element comprises first electrodes (20) on the expandable anchoring element; and
an inner sheath (30) slidably disposed within the outer sheath, the inner and outer sheath having distal portions fixed to one another, such that relative longitudinal movement of the inner and outer sheaths in opposite directions moves the expandable anchoring element between unexpanded and expanded positions;
a second therapy element adapted for positioning within a second blood vessel different from the first blood vessel, the second therapy element carried on a second shaft (24), wherein the second shaft includes:
a second outer sheath having a distal portion, and a second expandable anchoring element (26) on the distal portion, wherein the second therapy element comprises second electrodes (34) on the second expandable anchoring element, and
a second inner sheath slidably disposed within the second outer sheath, the second inner and second outer sheath having distal portions fixed to one another, such that relative longitudinal movement of the second inner and second outer sheaths in opposite directions moves the second expandable anchoring element between unexpanded and expanded positions;
wherein one of the first and second shafts is slidably received within a lumen of the other of the first and second shafts; and
a stimulator configured to (a) energize the first therapy element within the first blood vessel to deliver therapy to a first nerve fiber disposed external to the first blood vessel and (b) energize the second therapy element within the second blood vessel to deliver sympathetic therapy to a second nerve fiber disposed external to the first blood vessel.

2. The system of claim 1, further including control means for controlling the stimulation in response to sensed heart rate and/or blood pressure of the patient.

3. The system of claim 1, wherein each of the first and second therapy elements is at least partially expandable to position the first and second therapy elements in contact with surrounding walls of the first and second blood vessels.

4. The system of claim 1, further including an expandable balloon (28) coupled to the second therapy element distal to the second electrodes.

## Patentansprüche

1. Neuromodulationssystem (10) zum Behandeln eines Patienten, das Folgendes umfasst:
ein erstes Therapieelement, das zum Positionieren in einem ersten Blutgefäß ausgestaltet ist, wobei das erste Therapieelement auf einem ersten länglichen Schaft (12) getragen wird, wobei der erste Schaft Folgendes beinhaltet:
eine äußere Hülle (32) mit einem distalen Teil und einem erweiterbaren Verankerungselement (18) auf dem distalen Teil, wobei das erste Therapieelement erste Elektroden (20) auf dem erweiterbaren Verankerungselement umfasst; und
eine innere Hülle (30), die gleitend innerhalb der äußeren Hülle angeordnet ist, wobei die innere und die äußere Hülle distale Teile haben, die aneinander befestigt sind, so dass durch eine relative Längsbewegung der inneren und äußeren Hülle in entgegengesetzte Richtungen das erweiterbare Verankerungselement zwischen nicht erweiterten und erweiterten Positionen bewegt wird;
ein zweites Therapieelement, das zum Positionieren in einem zweiten Blutgefäß ausgestaltet ist, das sich vom ersten Blutgefäß unterscheidet, wobei das zweite Therapieelement auf einem zweiten Schaft (24) getragen wird, wobei der zweite Schaft Folgendes beinhaltet:
eine zweite äußere Hülle mit einem distalen Teil und einem zweiten erweiterbaren Verankerungselement (26) auf dem distalen Teil, wobei das zweite Therapieelement zweite Elektroden (34) auf dem zweiten erweiterbaren Verankerungselement umfasst, und
eine zweite innere Hülle, die gleitend innerhalb der zweiten äußeren Hülle angeordnet ist, wobei die zweite innere und die zweite äußere Hülle distale Teile haben, die aneinander befestigt sind, so dass durch eine relative Längsbewegung der zweiten inneren und der zweiten äußeren Hülle in entgegengesetzte Richtungen das zweite erweiterbare Verankerungselement zwischen nicht erweiterten und erweiterten Positionen bewegt wird;
wobei der erste oder der zweite Schaft gleitend in einem Lumen des jeweils anderen aus ersten und zweiten Schaft aufgenommen ist; und
einen Stimulator, der so konfiguriert ist, dass (a) das erste Therapieelement im ersten Blutgefäß mit Energie versorgt wird, um die Therapie einer ersten Nervenfaser zuzuführen, die außerhalb des ersten Blutgefäßes angeordnet ist, und (b) das zweite Therapieelement im zweiten Blutgefäß mit Energie versorgt wird, um eine sympathische Therapie einer zweiten Nervenfaser zuzuführen, die außerhalb des ersten Blutgefäßes angeordnet ist.

2. System nach Anspruch 1, das ferner ein Steuermittel zum Steuern der Stimulation als Reaktion auf die/den erfasste(n) Herzfrequenz und/oder Blutdruck des Patienten beinhaltet.

3. System nach Anspruch 1, wobei das erste und das zweite Therapieelement jeweils wenigstens teilweise erweiterbar sind, um das erste und das zweite Therapieelement in Kontakt mit den umliegenden Wänden der ersten und zweiten Blutgefäße zu positionieren.

4. System nach Anspruch 1, das ferner einen ausdehnbaren Ballon (28) beinhaltet, der mit dem zweiten Therapieelement distal zu den zweiten Elektroden gekoppelt ist.

## Revendications

1. Système de neuromodulation (10) destiné au traitement d'un patient, comprenant:
un premier élément thérapeutique adapté pour être positionné à l'intérieur d'un premier vaisseau sanguin, le premier élément thérapeutique étant porté sur une première tige allongée (12), la première tige comportant:
une gaine externe (32) présentant une partie distale et un élément d'ancrage déployable (18) sur la partie distale, dans lequel le premier élément thérapeutique comprend des premières électrodes (20) sur l'élément d'ancrage déployable; et
une gaine interne (30) disposée de manière coulissante à l'intérieur de la gaine externe, les gaines interne et externe présentant des parties distales fixées l'une à l'autre, de telle sorte qu'un mouvement longitudinal relatif des gaines interne et externe dans des sens opposés déplace l'élément d'ancrage déployable entre des positions non déployée et déployée;
un second élément thérapeutique adapté pour être positionné à l'intérieur d'un second vaisseau sanguin différent du premier vaisseau sanguin, le second élément thérapeutique étant porté sur une seconde tige (24), la seconde tige comportant:
une seconde gaine externe présentant une partie distale, et un second élément d'ancrage déployable (26) sur la partie distale, dans lequel le second élément thérapeutique comprend des secondes électrodes (34) sur le second élément d'ancrage déployable, et
une seconde gaine interne disposée de manière coulissante à l'intérieur de la seconde gaine externe, la seconde gaine interne et la seconde gaine externe présentant des parties distales fixées l'une à l'autre, de telle sorte qu'un mouvement longitudinal relatif de la seconde gaine interne et de la seconde gaine externe dans des sens opposés déplace le second élément d'ancrage déployable entre des positions non déployée et déployée;
dans lequel l'une des première et seconde tiges est reçue de manière coulissante à l'intérieur d'une lumière de l'autre des première et seconde tiges; et
un stimulateur configuré pour (a) exciter le premier élément thérapeutique à l'intérieur du premier vaisseau sanguin pour délivrer une thérapie à une première fibre nerveuse disposée à l'extérieur du premier vaisseau sanguin et (b) exciter le second élément thérapeutique à l'intérieur du second vaisseau sanguin pour délivrer une thérapie sympathique à une seconde fibre nerveuse disposée à l'extérieur du premier vaisseau sanguin.

2. Système selon la revendication 1, comportant en outre un moyen de commande pour commander la stimulation en réponse à un rythme cardiaque et/ou une pression sanguine détectés du patient.

3. Système selon la revendication 1, dans lequel chacun des premier et second éléments thérapeutiques est au moins partiellement déployable pour positionner les premier et second éléments thérapeutiques en contact avec des parois périphériques des premier et second vaisseaux sanguins.

4. Système selon la revendication 1, comportant en outre un ballon gonflable (28) couplé au second élément thérapeutique distal aux secondes électrodes.
